# EUROPEAN PATENT SPECIFICATION

(11) **EP 0 427 385 B1**
(45) Date of publication and mention of the grant of the patent: **07.08.1996**
(21) Application number: 90309821.8
(22) Date of filing: 07.09.1990
(51) Int. Cl.: C12N 15/57, C12N 15/80

(54) **Gene expression unit comprising the promoter and the terminator as well as the gene of alkaline protease**
Aus Promoter, Terminator und dem Gen der alkalischen Protease bestehende Genexpressionseinheit
Unité d'expression de gène comprenant le promoteur, le terminateur et le gène de la protéase alcaline

(30) Priority: 08.09.1989 JP 231660/89
(43) Date of publication of application: 15.05.1991
(73) Proprietor: THE GREEN CROSS CORPORATION, Osaka-shi Osaka (JP); KIKKOMAN CORPORATION, Noda-shi (JP)
(72) Inventor: Murakami, Kohji, c/o Chuo Kenkyusho, Otani-2-chome, Hirakata-shi (JP); Ishida, Yutaka, c/o Chuo Kenkyusho, Otani-2-chome, Hirakata-shi (JP); Masaki, Atsushi, c/o Chuo Kenkyusho, Otani-2-chome, Hirakata-shi (JP); Kawabe, Haruhide, c/o Chuo Kenkyusho, Otani-2-chome, Hirakata-shi (JP); Arimura, Hirofumi, c/o Chuo Kenkyusho, Otani-2-chome, Hirakata-shi (JP); Tatsumi, Hiroki, 399, Noda, Noda-shi (JP); Murakami, Seiji, 399, Noda, Noda-shi (JP); Nakano, Eiichi, 399, Noda, Noda-shi (JP); Motai, Hiroshi, 399, Noda, Noda-shi (JP)
(74) Representative: Coleiro, Raymond

(56) References cited:
- EP-A- 0 238 023
- AGRICULTURAL & BIOLOGICAL CHEMISTRY, vol. 52, no. 7, 1988; H. TATSUMI et al., pp. 1887-1888
- MOLECULAR & GENERAL GENETICS, vol. 219, no. 1-2, October 1989; H. TATSUMI et al., pp. 33-38

## Description

The present invention relates to a novel alkaline protease promoter and an alkaline protease terminator derived from a yellow mold, especially yellow koji mold, a gene expression unit comprising the promoter and the terminator, and relates to a genome gene of alkaline protease.

The structure of alkaline protease gene derived from Aspergillus oryzae which is one of the yellow molds is unknown. To our knowledge, the gene itself has not yet been isolated.

Alkaline protease is a hydrolase which acts on protein or its partial hydrolysate to decompose the peptide bond and, has been widely used as a drug, and in refreshments, detergents, etc.

As a result of various investigations on Aspergillus oryzae-derived alkaline protease gene, the present inventors succeeded for the first time in isolating Aspergillus oryzae-derived alkaline protease gene and prepro type alkaline protease gene and determining their structures, based on which patent applications were filed (Japanese Patent Application Nos. 63-51777 and 63-170018). However, these genes are those derived from mRNA of alkaline protease. Any such publication disclosing the genome gene of alkaline protease and its production does not disclose its structure.

On the other hand, Escherichia coli, Bacillus subtilis, yeast, animal cells, etc. have been heretofore studied as hosts capable of producing substances using genetic engineering. However, each of these hosts involves advantages and disadvantages. In particular, in the case of producing protein containing a sugar chain or protein having a certain steric structure, Escherichia coli, Bacillus subtilis and yeast are not suitable as hosts. In animal cells, costs for cultivation are high and many problems are involved in practical production. Thus, attention has recently been brought to an expression system using a mold as host. For example, according to Upshall et al., Bio Technology, 5, 1301-1304 (1987), TPA (tissue-type plasminogen activator) which was not expressed in Escherichia coli or yeast in its active form is expressed in mold (Aspergillus nidulans) in its active form.

Under such circumstances, it has become significant to develop a promoter or terminator effectively functioning in the host-vector system of molds.

An object of the present invention is to obtain a genome gene of alkaline protease from yellow mold, analyze its structure to determine promoter and terminator domains of the genome gene and newly obtain from the genome gene a promoter and a terminator effectively functioning in the host-vector system of molds and provide a gene expression unit comprising the promoter and the terminator.

As a result of extensive investigations, the present inventors have newly obtained the genome gene of alkaline protease, analyzed its structure and succeeded in identifying the promoter domain present at the 5'-end of the genome gene and the terminator domain present at the 3'-end of the genome gene. The present invention has thus been accomplished.

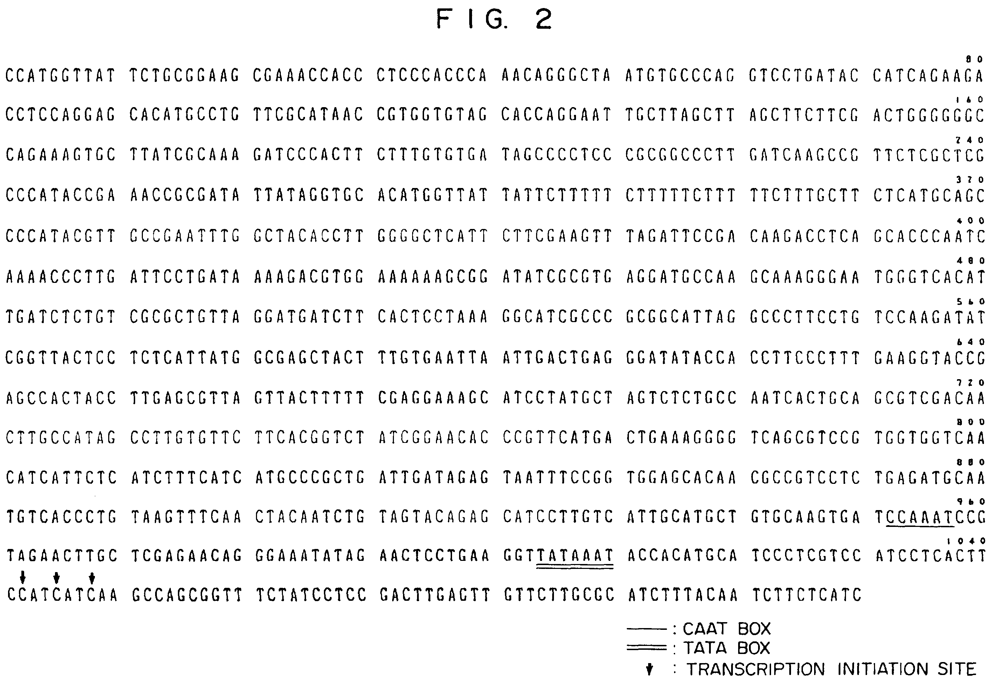

A first aspect of the present invention provides a plasmid comprising a promoter sequence derived from the *Aspergillus oryzae* alkaline protease genome gene, which sequence is as shown in Figure 2, or a functional equivalent thereof.

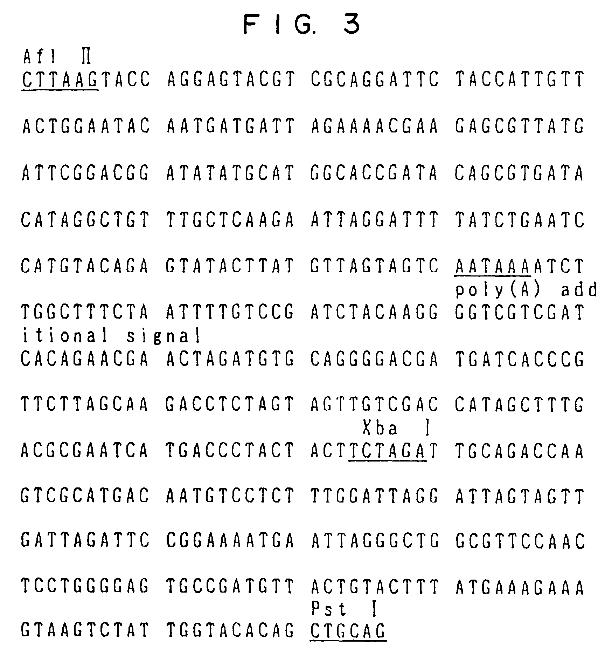

A second aspect of the present invention provides a plasmid comprising a terminator sequence derived from the *Aspergillus oryzae* alkaline protease genome gene, which sequence is as shown in Figure 3, or a functional equivalent thereof.

A third aspect of the present invention provides a gene expression unit comprising the alkaline protease promoter and the alkaline protease terminator.

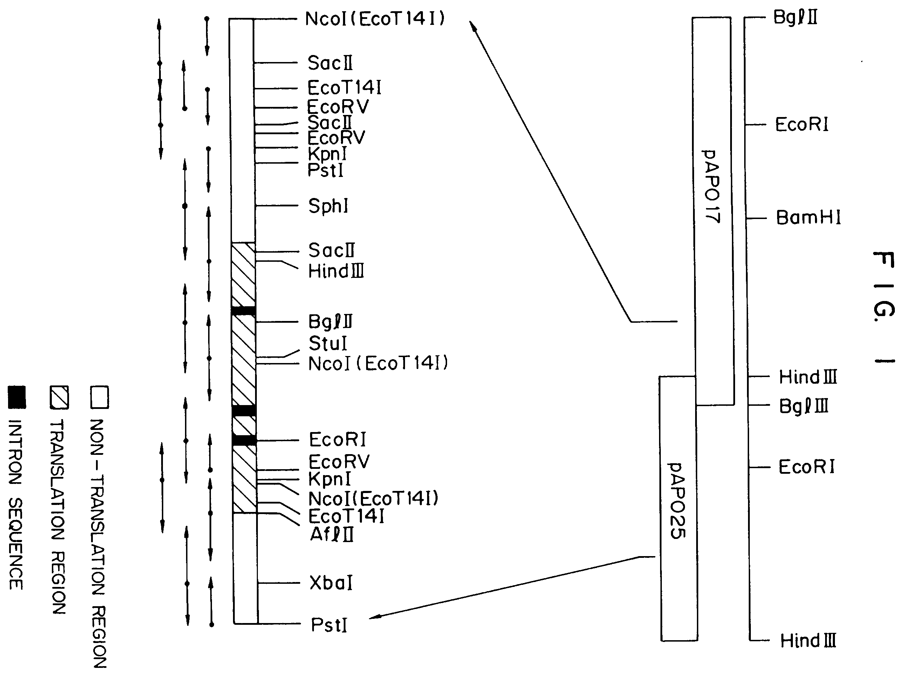

A fourth aspect of the present provides a plasmid comprising a genome gene of alkaline protease derived from *Aspergillus oryzae,* the gene having the restriction enzyme map shown in Figure 1.

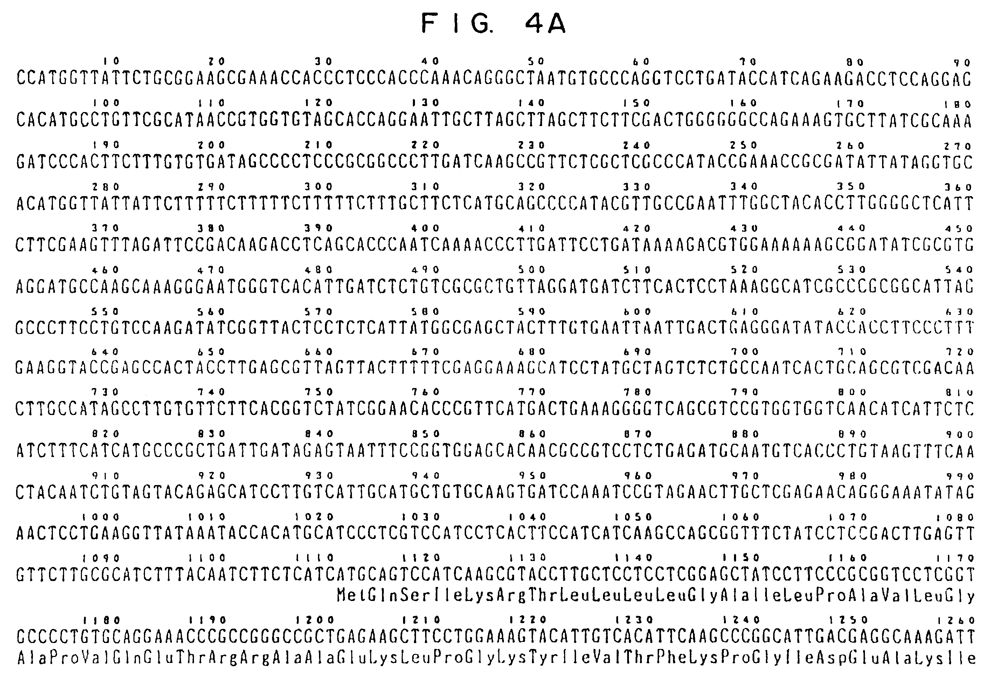

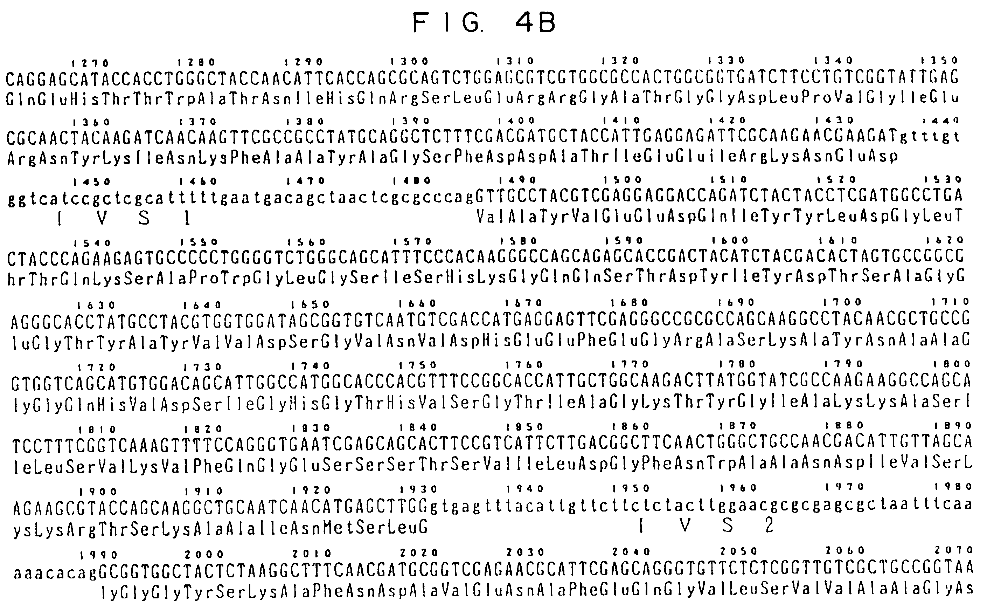

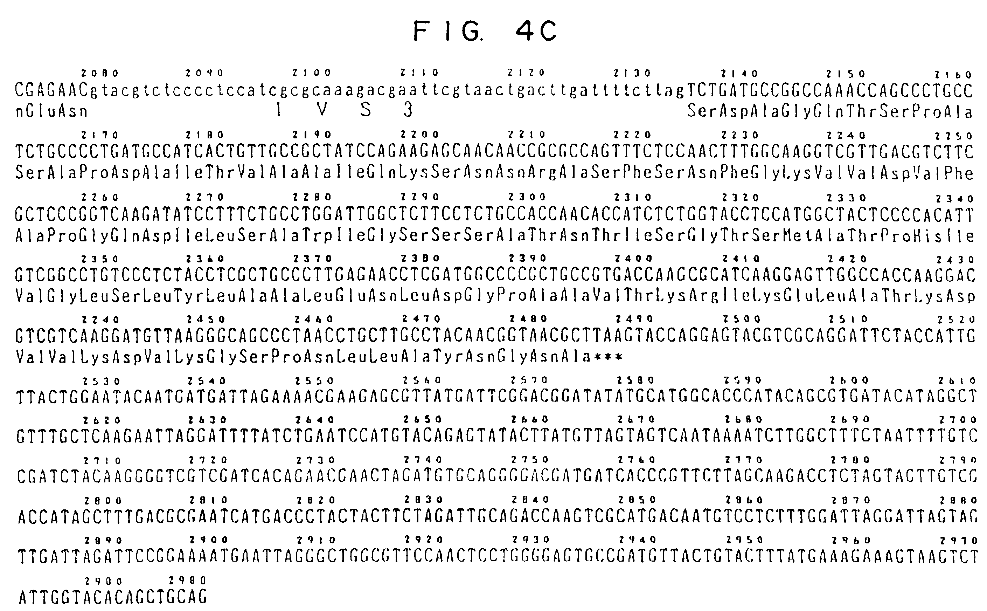

A fifth aspect of the present invention provides a plasmid comprising a genome gene of alkaline protease derived from *Aspergillus oryzae* and having the nucleotide sequence shown in Figure 4.

The invention thus enables determination of the structure of a genome gene of alkaline protease from yellow mold, which gene contains domains of the alkaline protease promoter and the alkaline protease terminator and containing 3 intron sequences.

In the accompanying drawings:

Fig. 1 shows a restriction enzyme map of genome gene of alkaline protease from yellow mold.

Fig. 2 shows a nucleotide sequence of genome gene of yellow mold alkaline protease containing yellow mold alkaline protease promoter at the 5'-end.

Fig. 3 shows a nucleotide sequence of genome gene of yellow mold alkaline protease containing yellow mold alkaline protease terminator at the 3'-end.

Fig. 4 shows the entire nucleotide sequence of genome gene of yellow mold alkaline protease.

Fig. 5 shows procedures for ligating alkaline protease genome genes.

Fig. 6 shows procedures for constructing expression vector pAP045.

Hereinafter embodiments of the present invention are described in detail.

The genome gene of alkaline protease and the promoter and terminator in accordance with the present invention are prepared as follows.

Chromosomal DNA is extracted from yellow mold (Aspergillus oryzae) by known methods (for example, Oakley et al., Gene, 61, 385-399 (1987)). The extracted chromosomal DNA is partially digested with a suitable restriction enzyme. After introduction of the digested gene into a plasmid vector (e.g., pUC19, etc.), host (e.g., Escherichia coli strain JM109, HB101, etc.; manufactured by Takara Shuzo Co., ltd.) is transformed with the vector to obtain a genomic library. By screening using a probe (e.g., cDNA probe, a synthesis probe, etc.), positive clone is obtained and the desired plasmid DNA is recovered. After digesting the plasmid DNA with a suitable restriction enzyme, the digestion product is introduced into a plasmid vector (e.g., pUC19, etc.) to transform therewith a host (e.g., Escherichia coli strain JM109 HB101). Thus, the desired clone is obtained.

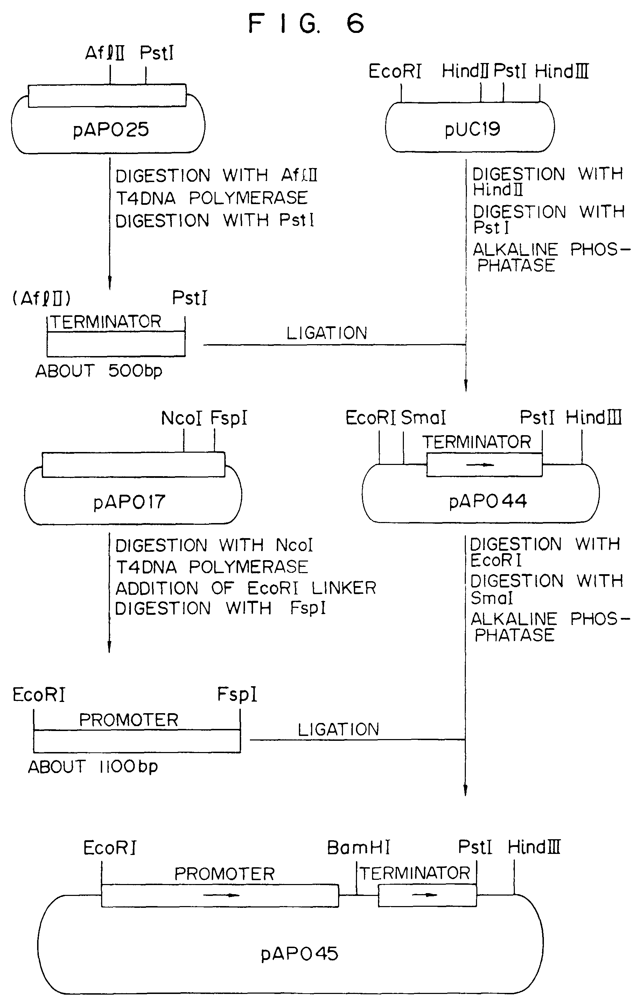

A plasmid in which a gene fragment containing the promoter of the present invention has been incorporated is pAP017 (cf. restriction enzyme map in Fig. 6). A plasmid in which a gene fragment containing the terminator of the present invention has been incorporated is pAP025 (cf. restriction enzyme map in Fig. 6).

The nucleotide sequence of DNA was analyzed by known methods (for example, the Maxam-Gilbert method or the dideoxy method).

The thus obtained genome gene of alkaline protease from yellow mold, the structure of which was determined as described above, has a restriction enzyme map shown in Fig. 1 and a nucleotide sequence shown in Fig. 4. In addition to the promoter and terminator domains utilised in the present invention, the genome gene also has 3 intron sequences, which are shown by IVS 1 to 3 in the figures.

The promoter of the present invention is present at the 5' site of the genome gene and also has 1110 bp DNAs. The nucleotide sequence is shown in Fig. 2 and corresponds to nucleotide No. 1 (C) to 1110 (C) in the nucleotide sequence of genome gene shown in Fig. 4.

In addition to DNA sequence shown in Fig. 2, plasmids of the present invention may also include other DNA sequences having the function equivalent to that of the promoter, such as DNA sequence having a nucleotide sequence partly different from the nucleotide sequence shown in Fig. 2, DNA sequence containing a part of the nucleotide sequence shown in Fig. 2 and DNA sequence containing at least the nucleotide sequence described above.

The terminator present in a plasmid of the present invention is present at the 3' site of the genome gene and has 530 bp DNAs. The nucleotide sequence is shown in Fig. 3 and corresponds to nucleotide No. 2458 (G) to 2988 (G) in the nucleotide sequence of genome gene shown in Fiq. 4.

In addition to DNA shown in Fig. 3, plasmids of the present invention may also include other DNA sequences having the function equivalent to that of the terminator, such as DNA sequence having a nucleotide sequence partly different from the nucleotide sequence shown in Fig. 3, DNA sequence containing a part of the nucleotide sequence shown in Fig. 3 and DNA sequence containing at least the nucleotide sequence described above.

By ligating the promoter or terminator with a suitable plasmid vector, the promoter or terminator may be utilized as an expression vector in yellow mold.

These promoter and terminator utilised in the present invention may be used in one set as a gene expression unit.

By introducing a gene coding for urokinase, hepatitis B antigen, human serum albumin, interferon α or interferon γ or a gene coding for its derivative into the resulting expression vector, further transforming therewith hosts such as Aspergillus oryzae, Aspergillus niger, Aspergillus awamori, Aspergillus sojae, etc. and culturing the transformants, the desired compounds can be obtained.

According to the present invention, the alkaline protease promoter and terminator, and the gene expression unit comprising the promoter and terminator as well as the genome gene of alkaline protease can be newly provided. In particular, the promoter and terminator, and the gene expression unit comprising the promoter and terminator can be used to construct an expression vector useful for molds such as Aspergillus oryzae, Aspergillus niger, Aspergillus awamori, Aspergillus sojae, etc., by ligating with a suitable plasmid vector. The present invention thus greatly contributes to production of useful substances using the host-vector system of molds.

Hereafter embodiments of the present invention are described in more detail with reference to the Examples below.

### Examples

### 1. Preparation of chromosomal DNA from Aspergillus oryzae

To extract chromosomal DNA from Aspergillus oryzae, the following procedures were carried out. Firstly, one platinum ear loop of Aspergillus oryzae strain ATCC 20386 was taken from slant and inoculated on 50 ml of YPS medium (1% yeast extract, 2% Bactopeptone and 2% soluble starch) followed by incubation at 30°C for 2 days. The culture broth was further inoculated on 500 ml of YPS medium charged in an Erlenmeyer's flask of 3 liter volume. Incubation was carried out at 30°C for one day. The hyphae were collected by filtering through 3 laminated sheets of gauze The collected cells were washed twice with 20 mM EDTA solution (pH 8.0). After washing further once with extraction buffer (100 mM Tris-hydrochloride, pH 8.0, 10 mM EDTA, 1% sodium lauryl sulfate), water was removed through filter paper. The wet cells were mixed with the same weight of sterilized sea sand B (manufactured by Nacalai tesque Co., Ltd.). The mixture was homogenized in a mortar for about 5 minutes. To the homogenate was added 60 ml of the extraction buffer (supra) and, the mixture was again homogenized for further 5 minutes. The homogenized cells were centrifuged in a centrifuging tube. The supernatant was transferred to a new centrifuging tube. RNase A (manufactured by Sigma Co.) was added to the supernatant in a final concentration of 5 µg/ml to react at 60°C for 20 minutes. 1/6-Fold volume of 3 M potassium acetate solution was added to the reaction mixture. After allowing to stand at room temperature, centrifugation was performed and the supernatant was transferred to a new, sterilized centrifuging tube. The supernatant was extracted twice with the same volume of TE-saturated phenol (equilibrated with the same volume of a solution containing 100 mM Tris-hydrochloride and 10 mM EDTA, pH 8.0) and then 3 times with the same volume of ether. To the solution after the extraction, 2-fold volume of ethanol was slowly added so as not to trouble the interface and the chromosomal DNA formed at the interface was wound around a Pasteur pipette. The wound DNA was rinsed, in sequence, with 70% ethanol, 90% ethanol and 100% ethanol. After drying, the DNA was dissolved in TE buffer (10 mM Tris-hydrochloride, 1 mM EDTA, pH 8.0).

### 2. Synthesis of probe

Generally in the case of cloning genomic gene using cDNA, hydridization is often performed using cDNA itself as a probe. However, where cDNA is used as a probe under a situation that the desired gene forms a family or has a pseudogene, its specificity becomes low. Therefore, for cloning of alkaline protease gene, oligonucleotide synthesized based on the non-translation region of the cDNA sequence (Japanese Patent Application No. 63-170018) at the 5′-and 3′-ends was used as a probe. The sequence of synthesized oligonucleotide is shown below.

AP-23: 5' GCG CAA GAA CAA CTC AAG TCG GAG GAT AGA 3'
AP-24: 5' CAT GTA CAG AGT ATA CTT ATG GTA GTA GTC 3'

AP-23 is an oligonucleotide having a size of 30 nucleotides (30-mer) which was designed based on the sequence of alkaline protease cDNA (Japanese Patent Application No. 63-17018) in the non-translation region at the 5'-end. AP-24 is a 30-mer oligonucleotide which was designed based on the sequence in the non-translation region at the 3'-end. These oligonucleotides were synthesized with a DNA synthesizer (381A) (manufactured by Applied Biosystems Co., ltd.), using the reagents and method indicated by the manufacturer. Radioactive label of the synthetic oligonucleotides was made using [γ-³²P]ATP (manufactured by Amersham Co., Ltd.) and T4 polynucleotide kinase (manufactured by Takara Shuzo Co., Ltd.).

### 3. Southern hydridization

Using the synthetic oligonucleotide probe prepared in procedure 2. above, the chromosomal DNA of Aspergillus oryzae ATCC 20386 was analyzed for alkaline protease gene, in accordance with the Southern hybridization method. Firstly, the chromosomal DNA of Aspergillus oryzae was digested with several restriction enzymes (for example, BamH I, Eco RI, etc.). Thereafter, isolation was performed by agarose gel electrophoresis. After the electrophoresis, DNA was subjected to blotting onto a nitrocellulose filter by the Southern transfer method. The Southern transfer method was performed according to the method of Maniatis et al., Molecular Cloning, A Laboratory Manual, Cold Spring Harbor Laboratory, Cold Spring Harbor, NY (1982). Hybridization was performed at 42°C In 6 x SSC (0.9 NaCl, 0.09 M trisodium citrate), 0.5% sodium lauryl sulfate, 5 x Denhardt solution (0.1% Ficoll, 0.1% polyvinylpyrrolidone, 0.1% bovine serum albumin), 0.01 M EDTA (pH 8.0), and a solution of 100 µg/ml transfer RNA. The probe was added in a concentration of about 1.0 x 10⁷ cpm/ml. Washing was carried out at 45°C with 6 x SSC and 0.5% sodium lauryl sulfate solution 3 times. After air-drying the filter, the filter was applied to an X-ray film (manufactured by Fuji Photo Film Co., Ltd., RX) at -80°C to obtain autoradiogram. As the result, a single and clear band was obtained respectively with both probes of AP-23 and AP-24, for the digestion products of Aspergillus oryzae chromosome with restriction enzymes.

### 4. Preparation of library

As the result of Southern hybridization described above, a band of about 6.5 kb was noted with the digestion product of Aspergillus oryzae-derived chromosomal DNA with Bgl II in the case of using AP-23 as the probe and a band of about 4.5 kb was noted with the digestion product with Hind III in the case of using AP-24. Bgl II and Hind III are restriction enzymes present in alkaline protease cDNA. It is thus considered that cloning of DNA fragments obtained with both restriction enzymes would cover cloning of alkaline protease gene over almost the entire region. Therefore, genomic library of Aspergillus oryzae using these restriction enzymes was prepared. Firstly, about 200 µg of the chromosomal DNA of Aspergillus oryzae prepared in 1, above was digested at 37°C with 500 units each of Bgl II and Hind III (both manufactured by Takara Shuzo Co., Ltd.) overnight. After 0.8% agarose gel electrophoresis (at 30 V overnight), DNA fragment having a size of 5.0 to 7.0 kb (kilobase) and DNA fragment having a size of 3.0 to 5.0 kb were extracted and purified from the Bgl II digestion product and the Hind III digestion product, respectively, based on the size of a molecular weight marker simultaneously subjected to electrophoresis. Extraction and purification from agarose gel can be performed by the method of Maniatis et al. (supra). On the other hand, after digestion with BamH I which formed the same cohesive end as with Bgl II or digestion with Hind III using pUC19 as the vector, the terminus was dephosphorylated with alkaline phosphatase (manufactured by Takara Shuzo Co., Ltd.) to prevent self ligation.

The DNA fragment of 5.0 to 7.0 kb as the Bgl II digestion product described above was mixed with pUC19 previously digested with BamH I, and the DNA fragment of 3.0 to 5.0 kb as the Hind III digestion product was likewise mixed with pUC19 previously digested with Hind III. Using "Ligation Kit"(manufactured by Takara Shuzo Co., Ltd.), ligation was performed. Competent cells (manufactured by Takara Shuzo Co., Ltd.) of Escherichia coli strain HB101 were transformed using the resulting mixture. A part of the transformed Escherichia coli was subjected to plating on L agar medium (1% Bacto-trypton, 0.5% yeast extract, 1% NaCl, 1.5% agar) supplemented with 50 µg/ml of ampicilin for determining frequency of transformation. The balance was supplemented with 50-fold volume of L-broth (1% Trypton, 0.5% yeast extract, 1% NaCl) supplemented with 50 µg/ml of ampicilin and cultured at 37°C overnight to amplify the colony. The colony was then stored at -80°C as a genomic library of Aspergillus oryzae.

### 5. Colony hybridization

Colony hybridization was performed as described below. Firstly, the genomic library of Aspergillus oryzae was applied to plating in about 10,000 clones per plate having a diameter of 150 mm, followed by incubation at 37°C overnight. As medium for the plate, L agar medium supplemented with 50 µg/ml of ampicillin was used. After the incubation, a nylon filter (manufactured by NEN, Colony/Plaque Screen) was gently placed on the agar to transfer the colony onto the nylon filter. The nylon filter onto which the colony had been transferred was lyzed by immersing the same in 0.5 N NaOH to denature DNA. The nylon filter was then immersed in 1 M Tris-hydrochloride (pH 7.5) solution to neutralize. After drying, the denatured DNA was fixed. The thus prepared filter was placed in a vinyl bag and hybridization was performed under the same conditions as in the Southern hybridization. As the result, several clones hybridizing with probes of AP-23 or AP-24 were obtained. From these clones, plasmid DNA was extracted by the alkaline-SDS method (Maniatis, supra) and its nucleotide sequence was determined by the dideoxy method. The nucleotide sequence coincided with the cDNA sequence of alkaline protease.

Plasmid carrying the Bgl II fragment of about 6.5 kb capable of hybridizing with the AP-23 probe and plasmid carrying the Hind III fragment of about 4.5 kb capable of hybridizing with the AP-24 probe were named pAP017 and pAP025, respectively. Using these plasmids, the following analysis was conducted.

### 6. Acquisition of genome gene of alkaline protease and determination of its DNA sequence

pAP017 and pAP025 obtained in procedure 5, above were digested with several restriction enzymes. Then, their fragment patterns were observed by agarose gel electrophoresis to prepare a restriction enzyme map. The results are shown in Fig. 1. The gene fractions contained in the two plasmids were overlapping between the Hind III site and the Bgl II site and almost the entire region of alkaline protease gene could be cloned by the plasmids. Thus, the DNA sequence was determined using appropriate restriction enzyme sites, as shown in Fig. 2. For determination of the DNA sequence, the dideoxy method was used. "M13 Sequencing Kit" (manufactured by Takara Shuzo Co., ltd.) was used for the reaction and "Apparatus for Electrophoresis for Determination of DNA Nucleotide Sequence" (manufactured by Takara Shuzo Co., Ltd.) was used for electrophoresis. Comparison of the nucleotide sequence of genomic gene with the nucleotide sequence of alkaline protease cDNA (Japanese Patent Application No. 63-170018) reveals that intron sequence at 3 sites in total was present in the alkaline protease gene; i.e., one at its prepro region and two at its mature protein region.

### 7. Determination of transcription initiation site

In order to analyze the promoter domain of the cloned alkaline protease gene, its transcription initiation site was determined. Known methods for determination of the transcription initiation site are the S1 mapping method and the primer extension method. In this run, the primer extension method was used. As the primer, oligonucleotide synthesized based on a reverse chain of DNA encoding 12 to 17 amino acids (within the preregion) of alkaline protease protein was used. The sequence of oligonucleotide is shown below.

AP-26: 5' CGC GGG AAG GAT AGC TCC 3'

The sequence of AP-26 was synthesized using "DNA Synthesizer (381A)" manufactured by Applied Biosystems Co., Ltd. The synthesized oligonucleotide was purified using "Oligonucleotide Purification Cartridge" manufactured by Applied Biosystems Co., Ltd. The terminal labeling of AP-26 was performed using [γ-³²P] ATP and T4 polynucleotide kinase. The reaction was carried out at 37°C for an hour in a solution of 50 mM Tris-hydrochloride (pH 8.0), 10 mM MgCl₂ and 10 mM dithiothreitol (DTT). [γ-³²P] ATP which was not used for the labeling was removed using "NENSORB^{TM}20" column (manufactured by NEN). By the foregoing procedures, oligonucleotide having radioactivity of about 10⁷ cpm per 1 µg DNA was obtained.

Next, about 3 µg of mRNA (Japanese Patent Application No. 63-170018) used as a template for cloning alkaline protease cDNA was mixed with about 10 ng of the AP-26 primer labeled with ³²P to make the volume 6 µl and, 1 µl of 10 x reverse transcription buffer (50 mM Tris-hydrochloride (pH 8.0), 500 mM KCl and 100 mM MgCl₂) was added thereto. After heat treatment at 70°C for 5 minutes, the mixture was allowed to stand at room temperature for 20 minutes to effect annealing of the primer and mRNA.

Next, 1 µl of 10 mM DTT, 20 mM dNTP (equimolar mixture of dATP, dGTP, dCTP and dTTP), 0.5 µl of ribonuclease inhibitor (117 units/µl, manufactured by Takara Shuzo Co., Ltd.) and 0.5 µl of reverse transcriptase (manufactured by Takara Shuzo Co., Ltd., 22 units/µl) were added to the solution. After reacting at 42°C for an hour, 10 µl of formamide solution (95% formamide, 0.1% xylene cyanol and 0.1% bromophenol blue) was added to the reaction mixture to terminate the reaction.

Then, 1 to 3 µl of the reaction solution obtained above was applied onto 6% acrylamide-urea gel. At the same time, the solution obtained by the dideoxy reaction using pAP017 as a template and AP-26 as a primer was applied to the same gel as a size marker. As a result, 3 bands were noted with the reaction subjected to the primer extension and 3 transcription initiation sites were present in the alkaline protease gene. The results are shown in Fig. 2. The sequence TATAAAT which is considered to be a so-called TATA box is present at the upstream by 30 to 40 nucleotides from the uppermost stream of the transcription initiation site thus determined. At a position upstream by 80 to 90 nucleotides, the sequence CCAAAT which is considered tobea CAAT box is present.

### 8. Ligation of alkaline protease genome gene

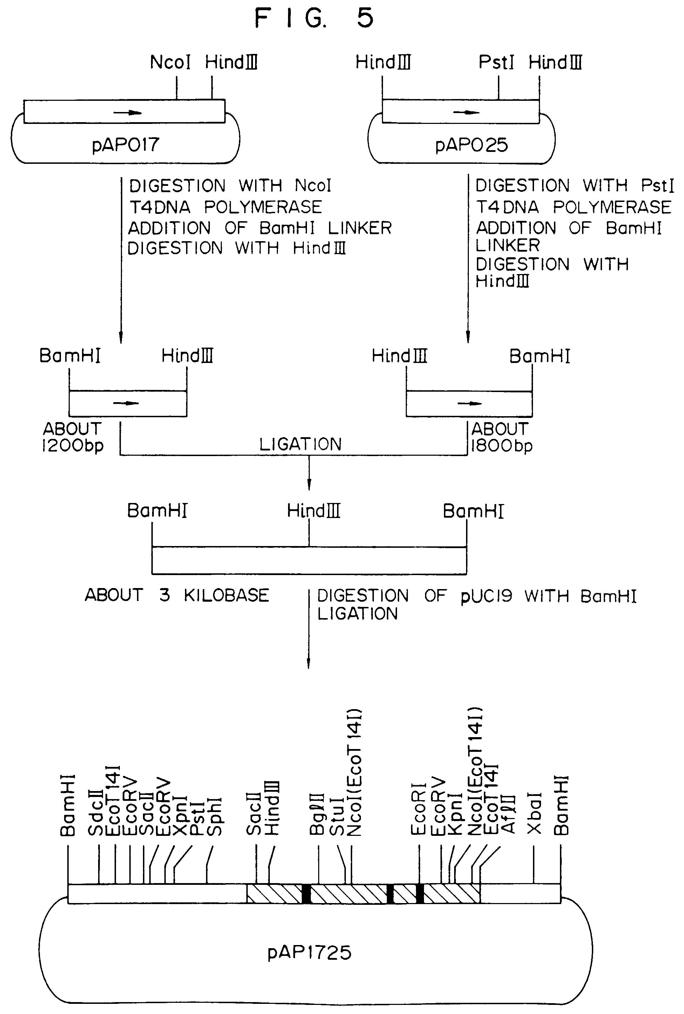

To ligate the alkaline protease genome genes cloned to the two DNA fragments (pAP017 and pAP025), the procedures shown in Fig. 5 were performed. Firstly, about 50 µg of pAP017 was digested with Nco I (manufactured by Takara Shuzo Co., Ltd.) and the terminus was rendered blunt, using "DNA Blunting Kit" (manufactured by Takara Shuzo Co., Ltd.). With the digested fragment was mixed 5 µg of BamH I linker (manufactured by Takara Shuzo Co., Ltd.). Using "DNA Ligation Kit" (manufactured by Takara Shuzo Co., Ltd.), both were ligated with each other. After purification by ethanol precipitation, DNA was digested with BamH I and Hind III (manufactured by Takara Shuzo Co., Ltd.). The DNA mixture was isolated by 1% agarose gel electrophoresis. The DNA fragment of about 1200 bp containing the 5'-site of alkaline protease genome gene was recovered and purified from the gel. Recovery of DNA from the gel can be performed according to the method of Maniatis et al. (supra). On the other hand, after digesting about 50 µg of pAP025 with Pst I (manufactured by Takara Shuzo Co., Ltd.), the terminus was rendered blunt, using "DNA Blunting Kit" (manufactured by Takara Shuzo Co., Ltd.). Likewise, 5 µg of BamH I linker was mixed with the digested fragment. Using "DNA Ligation Kit" (manufactured by Takara Shuzo Co., Ltd.), ligation was performed. After the ligation, purification was carried out by ethanol precipitation and DNA was digested with BamH I and Hind III.

The DNA digestion product was isolated by 1% agarose gel electrophoresis. The DNA fragment of about 1800 bp containing the 3'-site of alkaline protease genome gene was recovered and purified from the gel.

About 3 µg of the aforesaid 5'-site DNA fragment (about 1200 bp, BamH I/Hind III fragment) and about 3 µg of the 3′-site DNA fragment (about 1800 bp, BamH I/Hind III fragment) of alkaline protease were mixed with each other. After ligation by "DNA Ligation Kit", digestion was performed with BamH I. The digested DNA was isolated by 1% agarose gel electrophoresis and the DNA fragment having the desired size (about 3 kb) was recovered and purified. After the DNA fragment containing this alkaline protease genome gene was mixed with the BamH I-digested pUC19 to ligate them, the ligation product was transferred to competent cells of Escherichia coli strain JM109 (manufactured by Takara Shuzo Co., ltd.). A transformant which acquired ampicillin resistance was screened to obtain clone bearing the desired plasmid pAP1725. pAP1725 is DNA inserted with alkaline protease genome gene (about 3 kb) at the BamH I site of pUC19.

### 9. Preparation of expression vector

An expression vector using the promoter and terminator of alkaline protease gene was prepared by strategy shown in Fig. 6.

For isolating the terminator domain, 10 µg of pAP025 was digested with Afl II (manufactured by Takara Shuzo Co., Ltd.). Thereafter, the cleaved site was converted into the blunt end, using "DNA Blunting Kit" (manufactured by Takara Shuzo Co., Ltd.). After purification by ethanol precipitation, further digestion was performed with Pst I (manufactured by Takara Shuzo Co., Ltd.). The digested fragments were isolated by 1% agarose gel electrophoresis. Among them, DNA fragment (about 500 bp) in the terminator domain of alkaline protease was extracted and purified from the agarose gel. On the other hand, after pUC19 which may be used as a vector was digested with Pst I and Hinc II (manufactured by Takara Shuzo Co., Ltd.), the cleaved site was dephosphorylated with alkaline phosphatase (manufactured by Takara Shuzo Co., Ltd.). The cleaved vector (about 100 ng) was mixed with the terminator DNA fragment (about 500 bp) described above. After ligation of the mixture by "DNA Ligation Kit", Escherichia coli obtain JM109 (manufactured by Takara Shuzo Co., Ltd.) was transformed with the mixture. From the clone which acquired ampicillin resistance, plasmid DNA was extracted and screened to obtain the desired plasmid pAP044.

Next, for isolating the promoter domain, about 10 µg of pAP017 was digested with Nco I (manufactured by Takara Shuzo Co., Ltd.). Thereafter, the cleaved site was converted into the blunt end, using "DNA blunting Kit". After ethanol precipitation, DNA was mixed with 2 µg of Eco RI linker (manufactured by Takara Shuzo Co., Ltd.) followed by ligation using "DNA Ligation kit". The ligated DNA mixture was digested with Eco RI and Fsp I (manufactured by New England Biolab Co., Ltd.). The digested fragments were isolated by 1% agarose gel electrophoresis. Among the DNA fragments isolated, DNA fragment (about 1100 bp) containing the promoter domain of alkaline protease was extracted and purified from the agarose gel.

Then about 10 µg of plasmid pAP044 to which the terminator domain had been subcloned was digested with Eco RI and Sma I (manufactured by Takara Shuzo Co., Ltd.) followed by dephosphorylation by alkaline phosphatase. The deposphorylated product (about 100 ng) was mixed with the aforesaid promoter fragment (about 1100 bp, about 100 ng) to ligate them using "DNA Ligation Kit". Escherichia coli strain JM109 was transformed with the mixture. From the strain resistant to ampicillin, plasmid DNA was extracted and screened to obtain the desired plasmid pAP045. pAP045 is a plasmid ligated with the promoter and terminator of alkaline protease and is a vector capable of expression by inserting a heterologous gene into the BamH I site which is the unique recognition site.

## Claims

1. A plasmid comprising a promoter sequence derived from the *Aspergillus oryzae* alkaline protease genome gene, which sequence is as shown in Figure 2, or a functional equivalent thereof.

2. A plasmid comprising a terminator sequence derived from the *Aspergillus oryzae* alkaline protease genome gene, which sequence is as shown in Figure 3, or a functional equivalent thereof.

3. A plasmid according to claim 1 or claim 2 which is an expression vector.

4. A gene expression unit comprising a promoter sequence derived from the *Aspergillus oryzae* alkaline protease genome gene, which sequence is as shown in Figure 2, or a functional equivalent thereof, and a terminator sequence derived from the *Aspergillus oryzae* alkaline protease genome gene, which sequence is as shown in Figure 3, or a functional equivalent thereof.

5. An expression vector comprising a gene expression unit according to claim 4.

6. A plasmid comprising a genome gene of alkaline protease derived from *Aspergillus oryzae,* the gene having the restriction enzyme map shown in Figure 1.

7. A plasmid comprising a genome gene of alkaline protease derived from *Aspergillus oryzae* and having the nucleotide sequence shown in Figure 4.

## Patentansprüche

1. Plasmid, umfassend eine Promotorsequenz, die von dem Gen der alkalischen Protease aus dem Genom von Aspergillus oryzae stammt, wobei die Sequenz in Figur 2 gezeigt ist, oder ein funktionelles Äquivalent davon.

2. Plasmid, umfassend eine Terminatorsequenz, die von dem Gen der alkalischen Protease aus dem Genom von Aspergillus oryzae stammt, wobei die Sequenz in Figur 3 gezeigt ist, oder ein funktionelles Äquivalent davon.

3. Plasmid nach Anspruch 1 oder 2, das ein Expressionsvektor ist.

4. Genexpressionseinheit, umfassend eine Promotorsequenz, die von dem Gen der alkalischen Protease aus dem Genom von Aspergillus oryzae stammt, wobei die Sequenz in Figur 2 gezeigt ist, oder ein funktionelles Äquivalent davon, und eine Terminatorsequenz, die von dem Gen der alkalischen Protease aus dem Genom von Aspergillus oryzae stammt, wobei die Sequenz in Figur 3 gezeigt ist, oder ein funktionelles Äquivalent davon.

5. Expressionsvektor, umfassend eine Genexpressionseinheit nach Anspruch 4.

6. Plasmid, umfassend ein aus dem Genom von Aspergillus oryzae stammendes Gen der alkalischen Protease, das die in Figur 1 gezeigte Restriktionsenzymkarte hat.

7. Plasmid, umfassend ein aus dem Genom von Aspergillus oryzae stammendes Gen der alkalischen Protease, das die in Figur 4 gezeigte Nucleotidsequenz hat.

## Revendications

1. Plasmide comprenant une séquence de promoteur obtenue à partir d'un gène du génome de la protéase alcaline d'Aspergillus oryzae, laquelle séquence est telle que représentée dans la figure 2, ou un de ses équivalents fonctionnels.

2. Plasmide comprenant une séquence de terminateur obtenue à partir d'un gène du génome de la protéase alcaline d'Aspergillus oryzae, laquelle séquence est telle que représentée dans la figure 3, ou un de ses équivalents fonctionnels.

3. Plasmide selon la revendication 1 ou la revendication 2, qui est un vecteur d'expression.

4. Unité d'expression d'un gène comprenant une séquence de promoteur obtenue à partir d'un gène du génome de la protéase alcaline d'Aspergillus oryzae, laquelle séquence est telle que représentée dans la figure 2, ou un de ses équivalents fonctionnels, et une séquence de terminateur obtenue à partir d'un gène du génome de la protéase alcaline d'Aspergillus oryzae, laquelle séquence est telle que représentée dans la figure 3, ou un de ses équivalents fonctionnels.

5. Vecteur d'expression comprenant une unité d'expression d'un gène conforme à la revendication 4.

6. Plasmide comprenant un gène du génome de la protéase alcaline obtenu à partir d'Aspergillus oryzae, le gène ayant la carte d'enzyme de restriction représentée dans la figure 1.

7. Plasmide comprenant un gène du génome de la protéase alcaline obtenu à partir d'Aspergillus oryzae et ayant la séquence de nucléotides représentée dans la figure 4.
